# EUROPEAN PATENT SPECIFICATION

(11) **EP 0 506 818 B1**
(45) Date of publication and mention of the grant of the patent: **25.10.1995**
(21) Application number: 91901942.2
(22) Date of filing: 20.12.1990
(51) Int. Cl.: C07K 17/08, C12N 11/08

(54) **METHOD OF COVALENTLY BONDING PROTEINS TO HYDROPHILIC SURFACES**
VERFAHREN ZUR KOVALENTEN BINDUNG VON PROTEINEN AN HYDROPHILE OBERFLÄCHEN
PROCEDE DE LIAISON COVALENTE DE PROTEINES A DES SURFACES HYDROPHILES

(30) Priority: 29.12.1989 SE 8904397
(43) Date of publication of application: 07.10.1992
(73) Proprietor: BEROL NOBEL AB, S-444 85 Stenungsund (SE)
(72) Inventor: LINDMAN, Björn, S-223 63 Lund (SE); MALMSTEN, Martin, S-223 58 Lund (SE); HOLMBERG, Krister, S-431 39 Mölndal (SE); ANDREN, Carina, S-421 79 VA Frölunda (SE)
(74) Representative: Andersson, Rolf
(86) International application number: SE9000851
(87) International publication number: WO9109877

(56) References cited:
- EP-A- 0 187 391
- DIALOG INFORMATION SERVICES, WPI, File 351; Hitachi Chemical KK, AN 4926363
- DIALOG INFORMATION SERVICES, File 55, BIOSIS 85-91; M.B. STARK et al., AN 0020738475

## Description

The immobilisation of proteins to solid surfaces is an established technique within a number of applications, such as solid phase diagnostics, analysis with biosensors, affinity chromatography, extracorporeal therapy, and bio-organic synthesis. In all of these cases, the protein which may be a pure protein or a conjugate, such as a glycoprotein or a lipoprotein, is bonded to a solid surface, whereupon the biological activity of the protein is utilised for a specific purpose.

In solid phase diagnostics, an antibody is frequently immobilised to a plastic surface, usually consisting of polystyrene. When in contact with a body fluid, the immobilised antibody bonds any antigen that may be present. The antibody-antigen complex is then detected by means of a labelled antibody. The labelling may be in the form of a radioactive isotope, a fluorescent group, or an enzyme conjugate.

In extracorporeal therapy, a biologically active substance is bonded to a chamber through which the patient's blood is conducted. A current example of extracorporeal therapy is hemoperfusion across an immobilised immunostimulating substance. Interferons and interleukins are examples of such substances. Examples of diseases that can be treated by this technique are cancer and AIDS.

In bio-organic synthesis, use is made of enzymes for producing organic compounds. An appropriate use for bio-organic synthesis are lipid transformations, i.e. transforming a lipid, usually a triglyceride, into another. Most enzymes are expensive, and frequent reuse is necessary to ensure good process economy. In view hereof, the use of immobilised enzymes is of interest to most large-scale enzymatic processes.

As mentioned above, the immobilisation of proteins to both organic and inorganic surfaces is today a well-established technique (see chapter 4 in "Principles of Immobilization of Enzymes", Handbook of Enzyme Biotechnology, Second Edition, Ellis Horwood Limited, 1985), and it is possible to bond a large amount of protein to the surface while retaining adequate biologicial acitivity.

However, it has been found that most solid surfaces are so designed that they adsorb spontaneously proteins and other biomolecules. Adsorption in aqueous solution is promoted primarily by two types of physical forces, electrostatic attraction and hydrophobic interaction. Most surfaces are, at normal pH, negatively charged, but usually contain also hydrophobic domains. A protein usually has both positive, negative and hydrophobic seats, which means that a protein is attracted to most surfaces, on the one hand by electrostatic attraction between positive seats and negatively charged groups in the surface and, on the other hand, by hydrophobic interaction between hydrophobic domains of the protein and the surface. This is described in, for example, "Surface and Interfacial Aspects of Biomedical Polymers", Ed. J.D. Andrade, Plenum Press 1985, Vol. 2, p 81.

This nonspecific adsorption is an undesired phenomenon for the above-mentioned applications. In solid phase diagnostics, it results in an impaired sensitivity and a shorter life of the diagnostic kit. In both extracorporeal therapy and in bio-organic synthesis, spontaneous adsorption causes impaired activity and a shorter kit life.

One way of drastically reducing the adsorption of proteins and other biomolecules on solid surfaces is to provide the surfaces with a layer of an uncharged hydrophilic polymer. One example of a polymer that has been used for this purpose is polyethylene glycol (see C.-G. Gölander, "Preparation and Properties of Functionalised Polymer Surfaces", Dissertation, Royal Institute of Technology, Stockholm 1986), but other substances, such as polysaccharides, for example dextran, cellulose ethers and starch; polyvinyl alcohol; and neutral silica sol have also been used for this purpose.

By coating the surface with a layer of the uncharged hydrophilic polymer, both electrostatic attraction and hydrophobic interaction can be avoided. In this manner, the spontaneous adsorption of proteins can be reduced and occasionally almost entirely eliminated.

Hydrophilised surfaces of this type are of great interest to, inter alia, the above-mentioned applications of immobilised proteins. To covalently bond protein to such a surface, it is necessary to introduce into the hydrophilic layer reactive functional groups serving as anchoring points for the protein. However, it has proved extremely difficult to covalently bond protein to thoroughly hydrophilised surfaces, even if the surfaces contain a high concentration of reactive groups. The hydrophilic surface does not attract the protein. On the contrary, it acts as a repellent because it is energetically unfavourable for a protein in aqueous solution to approach such a surface. As a result, the amount of immobilised protein usually will be low, regardless of whether it is an antibody for solid phase diagnostics, an immuno-stimulating substance for extracorporeal therapy, or an enzyme for bio-organic synthesis.

The present invention has now shown that large amounts of protein can be immobilised to a hydrophilic surface. The protein-immobilised hydrophilic surface has a low spontaneous adsorption of undesired products. According to the invention, this is achieved by bonding protein to a hydrophilic surface which consists of a nonionic polymer immobilised in conventional manner on a carrier and having protein-reactive groups, the nonionic polymer having a cloud point of at least 5°C above the temperature at which the final product is to be used, and by covalently bonding the protein in per se known manner to the reactive groups of the nonionic polymer in a water-based reaction medium at a temperature which is more than 5°C below the cloud point of the polymer in the reaction medium. The basic principle of the invention is to utilise the unusual dependence on temperature exhibited by some nonionic water-soluble polymers. Thus, polyalkylene glycols and nonionic cellulose ethers exhibit a decreasing water solubility at elevated temperature. The mechanism behind this dependence on temperature has still not been fully explained, but it is assumed that the conformation of the ethylene oxide groups is changed in connection with an increase in temperature, making the ethylene oxide groups increasingly hydrophobic in character and thus less soluble in water. At a given temperature, the water solubility of the polymer is so low that the solution is phase-separated. This temperature is usually termed the cloud point of the solution. Polyalkylene glycols and cellulose ethers can both be produced with defined cloud points, and especially useful are the polymers whose cloud points lie within the range 10-100°C, preferably 30-50°C. The nonionic hydrophilic polymer shall be hydrophilic at the temperature at which the protein-coated surface is used. It is selected such that the cloud point is at least 5°C, preferably at least 10°C above the temperature at which the coated surface is used. A preferred protein immobilisation temperature is from 3°C below the flocculation temperature of the nonionic hydrophilic polymer in the reaction medium, to 50°C.

Examples of suitable polyalkylene glycols are those in which ethylene oxide and alkylene oxides having 3-4 carbon atoms, or tetrahydrofuran, are randomly distributed or distributed in blocks. Especially suitable are polyalkylene glycols having a molecular weight of 2,000-10,000 and containing one or more blocks of polyoxy propylene and polyoxy ethylene having a molecular weight of 300-3,000. Other types of suitable polyalkylene glycols are adducts of ethylene oxide in combination with higher alkylene oxides, or tetrahydrofuran with a dihydroxy or polyhydroxy compound, such as glycerol or pentaerythritol.

The cellulose ethers preferably have such a degree of polymerisation that a 1% aqueous solution thereof has a viscosity of 10-10,000 cP, preferably 30-5,000 cP, measured according to Brookfield, LV, 12 rpm at 20°C. They may comprise hydrophobic hydrocarbon groups, such as methyl, ethyl, propyl, butyl, benzyl and higher hydrocarbon groups having 8-24 carbon atoms, or polar hydroxyl groups, such as hydroxyethyl, hydroxypropyl and hydroxybutyl, or mixtures of hydrocarbon groups and polar groups. Examples of suitable cellulose ethers are methyl cellulose, ethyl cellulose, hydroxyethyl cellulose, hydroxypropyl cellulose, methyl hydroxyethyl cellulose, methyl hydroxypropyl cellulose, ethyl hydroxyethyl cellulose and benzyl ethyl hydroxyethyl cellulose. Alkyl hydroxyalkyl cellulose are preferred cellulose ethers.

To covalently bond the hydrophilic polymer to the carrier, and to covalently bond the protein to the hydrophilic polymer, reactive functional groups are introduced in conventional manner to serve as anchoring points. Examples of reactive groups that can be attached to the carrier are amino, carboxyl or hydroxyl groups with which the hydrophilic nonionic polymer or an activated form thereof can react. The hydrophilic polymer preferably comprises reactive groups, such as epoxy, tresylate, carbonyl imidazole and acyl azide groups capable of reacting with reactive groups on the carrier and with the protein which is normally bonded via one or more of its amino, thiol and/or phenolic hydroxyl groups. This immobilisation technique has been described in detail in, inter alia, the above-mentioned references "Surface and Interfacial Aspects of Biomedical Polymers", Ed. J.D. Andrade, Plenum Press 1985, Vol. 2, p 81, and C.G. Gölander: "Preparation and Properties of Functionalized Polymer Surfaces", Dissertation, Royal Institute of Technology, Stockholm 1986, which are hereby included as part of the description of this invention. In the event that the protein is a glycoprotein, bonding may occur to the aldehyde groups generated in the carbohydrate moiety by oxidation with, for example, sodium periodate. It is also possible to anchor the hydrophilic polymer to the solid polymer surface in conventional manner by physical adsorption.

The invention will be further illustrated by the following Examples.

### Example 1

Carboxyl groups were deposited on a polystyrene plate having the dimensions 2 x 2 cm by plasma polymerisation of acrylic acid on the surface. The carboxyl-functional surface was then treated with a 10% solution of diaminopropane in water in the presence of water-soluble carbodiimide (0.6%) at pH 4.5-5.0. The plate was rinsed with distilled water and treated with a 10% solution of a diepoxide of a block copolymer of ethylene oxide and propylene oxide having a molecular weight of 2,000 and a cloud point of 40°C at pH 9.5 for 15 h at 20°C.

A 10% carbonate buffer of pH 9.5 and containing 0.5 mg/ml immunoglobulin G (IgG) was heated to 40°C and contacted with epoxy- functional plates. The reaction was allowed to proceed for 2 h at 40°C.

As reference, use was made of on the one hand a polystyrene plate which had been hydrophilised in the same way as above, but in which the epoxide groups, before contact with the IgG solution at 40°C, had been removed by treatment with lye and, on the other hand, a polystyrene plate which had been hydrophilised as above, but in which the treatment with the IgG solution was carried out at 20°C instead of 40°C. In the latter case, the reaction time was 2 h and, alternatively, 8 h.

The amount of immobilised IgG was measured with enzyme-conjugated antibodies against IgG according to so-called ELISA (see, for instance, A. Voller and D.E. Bidwell in "Alternative Immunoassays" (W.P. Collins, Ed.) Chap. 6, Wiley, New York 1985). Evaluation was carried out by conventional spectroscopic technique.

| Sample | Adsorption at 495 mm |
|---|---|
| Surface without epoxide groups, 2 h | 0.167 |
| Bonding at 20°C, 2 h | 0.380 |
| Bonding at 20°C, 8 h | 0.405 |
| Bonding at 40°C, 2 h | 2.450 |

As will appear from the results, the amount of immobilised immunoactive protein is vastly increased when the bonding temperature is at the cloud point, as compared with a temperature of 20°C below this point. The increased immobilisation leads to improved sensitivity in immuno-diagnostics.

### Example 2

A PVC plate having the dimensions 12 x 8 cm and intended to form part of an extracorporeal chamber, was grafted with crotonic acid under irradiation with UV light of wavelength 320 nm and in the presence of benzophenone as initiator. The resulting carboxyl-functional surface was treated with a 10% solution polyethylene imine in water in the presence of a water-soluble carbodiimide (0.6%) at pH 4.5-5.0. The plate was then treated with a 10% solution of the bis(carbonyl imidazole) derivative of a block copolymer of ethylene oxide and propylene oxide having a molecular weight of 4,000 and cloud point of 38°C at pH 8.0 for 3 h at 20°C.

A 10% carbonate buffer of pH 8.0 and containing 50,000 U/ml γ-Interferon was contacted with carbonyl imidazole-functional hydrophilised PVC plates, and the reaction was allowed to proceed for 2 h and 8 h at the temperatures 20°C, 30°C and 40°C.

Peripheral mononuclear blood cells were isolated by gradient centrifugation on Lymphoprep (Ficoll solution). The cells were diluted to 1 · 10⁶/ml in the tissue culture medium RPMI 1640 with addition of 10% by weight of fetal calf serum (FCS) and 1% by weight of the antibiotic Penicillin streptomycin (PEST) and incubated on the γ-Interferon-immobilised plate for 7 days at 37°C and 5% CO₂.

As reference, use was also made of two plates which had only been hydrophilised as above, and to one of these plates free γ-Interferon was added directly into the cell culture at the beginning of the incubation.

Neopterin was used as a marker for the cell stimulation obtained. It is well known that mononuclear blood cells such as lymphocytes and macrophages, secrete neopterin when stimulated by, inter alia, γ-Interferon.

| Sample | Neopterin (nmol/1) |
|---|---|
| Hydrophilised surface with free γ-Interferon added | 83 |
| Hydrophilised surface without γ-Interferon | 5 |
| Bonding at 20°C, 2 h | 9 |
| Bonding at 20°C, 8 h | 11 |
| Bonding at 30°C, 2 h | 15 |
| Bonding at 30°C, 8 h | 20 |
| Bonding at 40°C, 2 h | 60 |
| Bonding at 40°C, 8 h | 62 |

The results indicate that biologically active γ-Interferon had been immobilised to a far higher degree when the immobilisation was carried out in accordance with the invention than when it had been carried out at lower temperatures. This circumstance may be utilised for cell stimulation, for example in extracorporeal therapy.

### Example 3

Silica (10 g) was reflux-boiled overnight in 150 ml of a 10% solution of 3-aminopropyl trimethoxy silane in toluene. The resulting aminopropyl silica was added to a solution consisting of 1 g periodate-oxidised ethyl hydroxyethyl cellulose having a cloud point of 42°C in 40 g aqueous solution of pH 7 in the presence of 0.5 g sodium cyanoborohydride. The reaction was allowed to proceed at 22°C under shaking for 16 h. The silica was filtered off and carefully washed with water.

To the aldehyde-functional cellulose-treated silica a 30 g aqueous solution was added, consisting of 33 mg/ml lipase and 0.5 g sodium cyanoborohydride. The reaction was allowed to continue for 16 h at pH 7, both at 20°C and at 40°C. Then the particles were washed alternatingly with carbonate buffer, pH 9.0, and acetate buffer, pH 4.0.

The amount of bonded protein was determined at 35 mg/g silica for the reaction at 40°C, and at 4 mg/g silica for the reaction at 20°C. The activity of the lipase from the 40°C reaction was measured in a trans-esterification reaction (incorporation of stearic acid in a triglyceride) and found to be 56% of the activity of free lipase. The same activity was found for lipase immobilised at 20°C, which means that the capacity of the preparation in accordance with the invention is about nine times higher than for the comparison preparation.

## Claims

1. A method of covalently bonding a protein to a hydrophilic surface consisting of a nonionic polymer immobilised in per se known manner on a carrier and exhibiting protein-reactive groups, **characterised** in that the nonionic polymer has a cloud point of at least 5° above the temperature at which the final product is to be used, and that the protein is covalently bonded in per se known manner to the reactive groups of the nonionic polymer in a water-based reaction medium at a temperature above 5°C below the cloud point of the polymer in the reaction medium.

2. Method as claimed in claim 1, **characterised** in that the nonionic hydrophilic polymer has a cloud point in the range 30-100°C, preferably 30-50°C.

3. Method as claimed in claim 1 or 2, **characterised** in that the nonionic hydrophilic polymer is a polyalkylene glycol compound.

4. Method as claimed in claim 1 or 2, **characterised** in that the nonionic hydrophilic polymer is a cellulose ether.

5. Method as claimed in claim 4, **characterised** in that the cellulose ether is an alkyl hydroxyalkyl cellulose having a viscosity of 30-5,000 cP, measured according to Brookfield, LV, 12 rpm at 20°C.

6. Method as claimed in any one of claims 1-5, **characterised** in that the nonionic hydrophilic cellulose ether is selected such that its cloud point is at least 10°C above the temperature at which the final product is to be used.

7. Method as claimed in any one of claims 1-6, **characterised** in that the hydrophilic polymer has epoxy, tresylate, carbonyl imidazole or acyl azide groups capable of reacting with reactive groups on the carrier and with the protein.

## Patentansprüche

1. Verfahren zur kovalenten Bindung eines Proteins an eine hydrophile Oberfläche, die aus einem nicht-ionischen Polymer besteht, das auf per se bekannte Weise auf einem Träger immobilisiert ist und Protein-reaktive Gruppen aufweist, dadurch gekennzeichnet, daß das nicht-ionische Polymer einen Trübungspunkt von mindestens 5°C oberhalb der Temperatur aufweist, bei der das Endprodukt verwendet werden soll, und daß das Protein auf per se bekannte Weise in einem Reaktionsmedium auf Wasserbasis bei einer Temperatur von mehr als 5°C unterhalb des Trübungspunktes des Polymers in dem Reaktionsmedium kovalent an die reaktiven Gruppen des nicht-ionischen Polymers gebunden wird.

2. Verfahren nach Anspruch 1, dadurch gekennzeichnet, daß das nicht-ionische hydrophile Polymer einen Trübungspunkt im Bereich von 30-100°C, vorzugsweise 30-50°C, aufweist.

3. Verfahren nach Anspruch 1 oder 2, dadurch gekennzeichnet, daß das nicht-ionische hydrophile Polymer eine Polyalkylenglykol-Verbindung ist.

4. Verfahren nach Anspruch 1 oder 2, dadurch gekennzeichnet, daß das nicht-ionische hydrophile Polymer ein Celluloseether ist.

5. Verfahren nach Anspruch 4, dadurch gekennzeichnet, daß der Celluloseether eine Alkylhydroxyalkylcellulose mit einer Viskosität von 30-5000 cP, gemessen nach Brookfield, LV, 12 UpM bei 20°C, ist.

6. Verfahren nach irgendeinem der Ansprüche 1-5, dadurch gekennzeichnet, daß der nicht-ionische hydrophile Celluloseether so ausgewählt ist, daß sein Trübungspunkt mindestens 10°C oberhalb der Temperatur liegt, bei der das Endprodukt verwendet werden soll.

7. Verfahren nach irgendeinem der Ansprüche 1-6, dadurch gekennzeichnet, daß das hydrophile Polymer Epoxy-, Tresylat-, Carbonylimidazol- oder Acylazid-Gruppen aufweist, die in der Lage sind, mit reaktiven Gruppen auf dem Träger und mit dem Protein zu reagieren.

## Revendications

1. Procédé de liaison covalente d'une protéine à une surface hydrophile consistant en un polymère non ionique immobilisé, de manière connue en soi, sur un support et présentant des groupes pouvant réagir avec les protéines, procédé caractérisé en ce que le polymère non ionique a un point de trouble situé à au moins 5°C au-dessus de la température à laquelle le produit final est à utiliser, et en ce que la protéine est liée par covalence, de manière connue en soi, aux groupes réactifs du polymère non ionique dans un milieu de réaction à base aqueuse, à une température située à plus de 5°C au-dessous du point de trouble du polymère dans le milieu de réaction.

2. Procédé tel que revendiqué à la revendication 1, caractérisé en ce que le polymère hydrophile non ionique a un point de trouble compris dans l'intervalle allant de 30 à 100°C et de préférence entre 30 et 50°C.

3. Procédé tel que revendiqué à la revendication 1 ou 2, caractérisé en ce que le polymère hydrophile non ionique est un composé de type polyalkylène glycol.

4. Procédé tel que revendiqué à la revendication 1 ou 2, caractérisé en ce que le polymère hydrophile non ionique est un éther de la cellulose.

5. Procédé tel que revendiqué à la revendication 4, caractérisé en ce que l'éther de la cellulose est une alkyl hydroxy alkyl cellulose ayant une viscosité de 30 à 5000 cP, mesurée selon Brookfield, LV, 12 tours par minute à 20°C.

6. Procédé tel que revendiqué dans l'une quelconque des revendications 1 à 5, caractérisé en ce que l'éther de cellulose hydrophile non ionique est choisi de façon que son point de trouble se situe à au moins 10°C au-dessus de la température à laquelle le produit final est à utiliser.

7. Procédé tel que revendiqué dans l'une quelconque des revendications 1 à 6, caractérisé en ce que le polymère hydrophile comporte des groupes époxyde, tresylate, carbonyle, imidazole ou azide d'acyle, capables de réagir avec des groupes réactifs présents sur le support et avec la protéine.
